# EUROPEAN PATENT SPECIFICATION

(11) **EP 3 017 036 B1**
(45) Date of publication and mention of the grant of the patent: **22.03.2017**
(21) Application number: 13762899.6
(22) Date of filing: 05.07.2013
(51) Int. Cl.: C12M 3/00, C12M 1/32, C12M 1/00

(54) **A DEVICE FOR CULTURING A BIOLOGICAL MATERIAL**
VORRICHTUNG ZUR KULTIVIERUNG EINES BIOLOGISCHEN MATERIALS
DISPOSITIF DE CULTURE D'UN MATÉRIEL BIOLOGIQUE

(43) Date of publication of application: 11.05.2016
(73) Proprietor: At Medical Ltd., 51230 Kaunas (LT)
(72) Inventor: PEDERSEN, Thomas William, DK-8660 Skanderborg (DK)
(74) Representative: Skov, Anders
(86) International application number: PCT/IB2013/055502
(87) International publication number: WO 2015/001397

(56) References cited:
- WO-A1-91/06624
- WO-A1-2013/004644
- WO-A2-2010/124663
- JP-A- 2003 289 850
- US-A1- 2011 104 730

## Description

### Field of the invention

The present invention relates in a first aspect to a device for culturing biological matter. In a second aspect the present invention relates to a kit-of-parts comprising a combination of a device of the first aspect of the present invention and an incubator. In a third aspect the present invention relates to use of a device according to the first aspect or to use of a kit-of-parts according to the second aspect for culturing biological matter. In a fourth aspect the present invention relates to a method for culturing a biologic material.

### Background of the invention

Within the field of culturing live biological matter it is well known to use culturing dishes for accommodating the biological material in which the biological material is allowed to grow in a growth medium or culture medium. Such dishes may be in the form of a simple cylindrical dish having a flat bottom, a relatively large diameter and a relatively small height. Such a dish is denoted a "petri dish" and is suitable for culturing biological material within a wide range of technologies.

However, in the field of in vitro fertilization in which live embryos are cultured in vitro for a few days following conception of an egg, it has been the practice for decades to apply a slightly more sophisticated design of the culture dish.

US 5,891,712 discloses such a culture dish. The culture dish of US 5,891,712 discloses a flat-bottomed dish comprising a plurality of circular concave wells embedded into the material of the dish in its interior. The wells may be arranged in different patterns, such as one central well having the remainder of wells concentrically arranged around the central well; or in two parallel rows of wells. US 5,891,712 does not disclose that any one well may have a different layout than any of the others on the same culture dish.

In the case of in vitro culturing of embryos it has recently become customized practice to employ a culture dish utilizing the so-called well-in-well principle. In a culture dish employing the well-in-well principle, the dish is formed of a material having a number of wells cut out in the material itself. Each well in its bottom additionally comprises a smaller well suitable to confine an egg or embryo within a rather confined space.

WO 2009/003487 A2 discloses such a well-in-well culture dish. The dish comprises a number of wells consisting of a depression (a well) having a diameter of 1 - 25 mm. Each depression (well) comprises in its center an indent (a well-in-well) consisting of a smaller depression having a diameter of approximately 0.2 mm and a depth of approximately 0.3 mm. A slope having an angle of 5 - 60° in relation to the horizontal direction is connecting the top rim of the indent with the bottom rim of the depression. A culture dish of WO 2009/003487 A2 having 12 such wells is exemplified. WO 2009/003487 A2 does not disclose that any one well-in-well may have a different layout than any of the others on the same culture dish.

In culturing live biological material, such as embryos or stem cells, it will be required in order to obtain optimum growth of the biological material to occasionally change the culture medium or growth medium used; either to refresh the same type of medium or to change the chemical composition of the medium.

In such a case when using the prior art culture dishes, the biological material will have to be carefully removed from each culture well and intermittently placed at a save place until the growth or culture medium has been changed. Such save place for accommodating the biological material during transfer of growth medium will often be a separate culture dish provided for this purpose. However such transfer to an external culture dish will impose various risks to the quality of the result of the work performed by the laboratory technician.

First of all, the transfer of a live biological material during transfer of medium will involve risk of contamination of the biological material.

Secondly, removing more entities of biological material originating from their respective locations in the wells of the culture dish to an external dish will involve the risk of intermixing the original location in the culture dish, resulting in a situation in which an entity of biological material may not be transferred back to its originally allocated well, but instead to another well of the same dish. Such a situation is clearly not optimal.

Accordingly, a need for an improved culture dish for culturing a biological material, such as an embryo or one or more stem cells, persists.

### Brief description of the invention

The above mentioned disadvantages are overcome by the present invention in its first, second, third and fourth aspect.

The present invention relates in its first aspect to a device for culturing biological matter, said device in the orientation intended for use comprises:
- a dish of a material, said dish having a top surface and an essentially flat, horizontal bottom surface;
wherein said dish comprising a number of well assemblies, said number of well assemblies being 3 or more;
wherein in respect of one or more of said well assemblies, preferably all well assemblies, said well assembly comprising a culture well and one or more associated washing wells,
wherein in respect of one or more of said well assemblies, preferably all well assemblies, said culture well and said one or more associated washing wells are grouped together;
wherein in respect of one or more of said well assemblies, preferably all well assemblies, said culture well comprises a culture well depression in said material, said culture well depression having a rim, wherein at least one part of said rim defines the beginning of a slope extending upwards, thus defining a culture well slope, said culture well slope ending in a well slope plateau;
wherein in respect of one or more of said well assemblies, preferably all well assemblies, said washing well comprises a washing well depression in said material, said washing well depression having a rim, wherein at least one part of said rim defines the beginning of a slope extending upwards, thus defining a washing well slope, said washing well slope ending in a well slope plateau;
wherein in respect of one or more of said well assemblies, preferably all well assemblies, said material at the position of the bottom of the culture well depression, and optionally also at the position of the bottom of the washing well depression, being transparent.

The device is characterized in that in respect of one or more of the well assemblies, preferably all well assemblies, said culture well slope comprises a number of protrusions, protruding from the surface of the culture well slope, said number of protrusions being present across the slope in at least a part thereof, preferably in a horizontal direction.

In case a commercial growth medium contains un-dissolved matter, the presence of such protrusions on the culture well slope may act as filter helping in an easy way to filter off any solid matter in handing and loading the growth medium.

The present invention relates in its second aspect to a kit of parts comprising one or more devices according to the first aspect of the present invention; and an incubator, wherein said incubator is configured to accommodate one or more of said devices.

The present invention relates in its third aspect to a use of a device according to the first aspect of the present invention; or of a kit of parts according to the second aspect of the present invention for culturing a biological material.

The present invention relates in its fourth aspect to a method of culturing a biological material, said method comprises:
- providing a device according to the first aspect of the present invention;
- arranging a biological material in the culture well depression of one or more of said culture wells;
- adding a growth medium to said culture well depressions;
- optionally covering said biological material and growth medium with an inert fluid, such as mineral oil;
- providing viable growth conditions to said biological material;
- allowing the biological material to grow for a predetermined time.

By providing one or more of the well assemblies with one or more washing wells in addition to the culture well allows a laboratory technician during change of culture medium or growth medium - in respect of each such well assembly - to intermittently transfer the biological material from the culture well to one of the washing wells associated to this specific culture well. Hence, no risk of imposing any external contamination is involved.

Also, the problem with the prior art culture dishes that during change of culture medium, the biological material may accidentally be returned to a culture well, which is not the culture well originally assigned to that specific biological material can be avoided because in the device of the first aspect of the present invention, any washing well is in close proximity to its associated culture well.

### Brief description of the figures

Fig. 1 shows in a perspective view a device according to the first aspect of the present invention for culturing a biological material.
Fig. 2 shows in a cross-sectional view details of the wells of a a device according to the first aspect of the present invention for culturing a biological material.
Fig. 3 shows in a plan view details of the wells of a device according to the first aspect of the present invention for culturing a biological material.
Fig. 4 a) - f) show various configurations of the culture well and the associated washing well(s) of a device according to the first aspect of the present invention for culturing a biological material.

### Detailed description of the invention

### The first aspect of the present invention

The first aspect of the present invention relates to a device for culturing a biological matter, said device in the orientation intended for use comprises:
- a dish of a material, said dish having a top surface and an essentially flat, horizontal bottom surface;
wherein said dish comprising a number of well assemblies, said number of well assemblies being 3 or more;
wherein in respect of one or more of said well assemblies, preferably all well assemblies, said well assembly comprising a culture well and one or more associated washing wells,
wherein in respect of one or more of said well assemblies, preferably all well assemblies, said culture well and said one or more associated washing wells are grouped together;
wherein in respect of one or more of said well assemblies, preferably all well assemblies, said culture well comprises a culture well depression in said material, said culture well depression having a rim, wherein at least one part of said rim defines the beginning of a slope extending upwards, thus defining a culture well slope, said culture well slope ending in a well slope plateau;
wherein in respect of one or more of said well assemblies, preferably all well assemblies, said washing well comprises a washing well depression in said material, said washing well depression having a rim, wherein at least one part of said rim defines the beginning of a slope extending upwards, thus defining a washing well slope, said washing well slope ending in a well slope plateau;
wherein in respect of one or more of said well assemblies, preferably all well assemblies, said material at the position of the bottom of the culture well depression, and optionally also at the position of the bottom of the washing well depression, being transparent.

The device is characterized in that in respect of one or more of the well assemblies, preferably all well assemblies, said culture well slope comprises a number of protrusions, protruding from the surface of the culture well slope, said number of protrusions being present across the slope in at least a part thereof, preferably in a horizontal direction.

Accordingly, the device according to the first aspect of the present invention comprises a dish having a number of well assemblies, wherein each well assembly comprises one culture well and a one or more washing wells. The culture well is intended for culturing a biological material, whereas the washing wells are intended for accommodating the biological material during exchange of growth medium of the biological material.

The device according to the first aspect of the present invention is especially well-suited for culturing embryos originating from an in vitro fertilization procedure or for culturing one or more stem cells, such as embryos or stemcells of mammal origin, such as of human origin. In the present description and in the appended claims, the term "in the orientation intended for use" shall be interpreted to mean an orientation in which the bottom horizontal surface is horizontally oriented and pointing downwards.

In the present description and in the appended claims, the term "plateau" shall be interpreted to mean an area in which a well slope ends. A plateau may thus be an area join two or more well slopes in the form of a platform or the like, or alternatively, a plateau may be merely a rim joining such two or more well slopes.

In a preferred embodiment of the device according to the first aspect of the present invention, the number of well assemblies is 4 - 30, such as 6 - 28, e.g. 8 - 26, such as 10 - 24, for example 12 - 22, such as 14 - 20, e.g. 16 - 18.

The device according to the first aspect of the present invention is especially well suited for culturing human embryos in an in vitro fertilization. In such a procedure a number of eggs is taking from a woman's ovaries and arranged on a culture disc such as the device according to the present invention's first aspect. For economic reasons, an in vitro fertilization procedure often involves subjecting the woman to a hormone treatment which will ensure that the woman will produce an extended number of ripened eggs ready for conception, compared to the situation in which no hormone treatment is provided. Upon a hormone treatment, usually 8 - 14 eggs will be retrieved from the woman's ovaries and these will be arranged in a culture disc with the view of culturing the conceived egg.

Accordingly, it will be appropriate to provide the device for culturing a biological material with a number of well assemblies which will be sufficient in most cases to allow allocation of a single device to a single woman's eggs. The above number of well assemblies has proven suitable for this purpose.

In one embodiment of the device according to the first aspect of the present invention, the number of washing wells in each specific well assembly being 1, 2, 3 or 4.

These numbers of washing wells associated with each culture well of one or more of the well assemblies have proven suitable when the device is for use for culturing an embryo.

In one embodiment of the device according to the first aspect of the present invention, more than half of the culture well depressions are arranged in such a way that they form a single straight line extending in an essentially horizontal direction.

In one embodiment of the device according to the first aspect of the present invention, all the culture well depressions are arranged in such a way that they form a single straight line extending in an essentially horizontal direction.

In one embodiment of the device according to the first aspect of the present invention, the number of culture well depressions arranged in a single straight line extending in an essentially horizontal direction is 3 - 20, such as 4 - 19, for example 5 - 18, such as 6 - 17, e.g. 7 - 16, for example 8 - 15, e.g. 9 - 14, such as 10 - 13 or 11 - 12.

By having the culture well depressions of a number of the well assemblies extend in an essentially straight line will make it possible to use the devices in an incubator which is provided with image capturing means, such as a camera, and in which the image capturing means are adapted to only move in one direction. By only allowing image capturing means of an incubator to move in one direction will maximize the quality of the images captured because less errors in focusing the image by the image capturing means will result.

In one embodiment of the device according to the first aspect of the present invention, the culture well depression in respect one or more of said well assemblies, preferably in respect of all well assemblies, is having an essentially conical shape, or an essentially spherical shape.

In one embodiment of the device according to the first aspect of the present invention, the washing well depressions(s) in respect of one or more well assemblies, preferably in respect of all well assemblies, is/are having an essentially conical shape or an essentially spherical shape.

It will be preferred that the culture well is clearly distinguishable from its associated washing well(s). This feature may be provided by making sure that the washing well(s) is/are having a different layout than the culture well; either in respect of shape or in respect of size, or both.

In one embodiment of the device according to the first aspect of the present invention, said culture well and said one or more associated washing wells, in respect of one or more of said well assemblies, preferably in respect of all well assemblies, are grouped together in a distinct group, such as being arranged more proximate to each other compared to any surrounding well.

In one embodiment of the device according to the first aspect of the present invention, the well assembly, in respect of one or more of said well assemblies, preferably all well assemblies, comprises two washing wells, wherein the two washing wells and the culture well share a common slope plateau, and wherein the culture well depression is arranged in a direction essentially opposite to the direction of the washing well depressions, when viewed from said common slope plateau.

Usually two washing wells associated with each culture well will be sufficient when culturing embryos. By grouping the wells of the well assemblies in a configuration as set out above, space optimization of the device may be achieved.

In one embodiment of the device according to the first aspect of the present invention, the direction from a culture well depression to said common slope plateau in respect of one or more of said well assemblies, preferably all well assemblies is opposite to the direction from a culture well depression to said common slope plateau in respect of an adjacent well assembly, thus forming a staggered configuration of well assemblies.

Such a configuration of the wells of the well assemblies has proven to optimize the space available of the device. Furthermore, such a configuration will make it possible to use the devices in an incubator which is provided with image capturing means, such as a camera, and in which the image capturing means are adapted to only move in one direction, in case the culture well depressions of the washing wells additionally being arranged in a straight line.

In one embodiment of the device according to the first aspect of the present invention, said dish comprises a dish identification tag.

In one embodiment of the device according to the first aspect of the present invention, one or more, preferably all well assemblies comprise a well assembly identification tag.

In one embodiment of the device according to the first aspect of the present invention, one or more of said all culture wells, preferably all culture wells, comprise a culture well identification tag.

Providing the device with one or more identification tags relating to identifying the device, the well assemblies or the individual wells will help minimizing errors relating to mixing up different biological materials by the laboratory technician handling the device.

In one embodiment of the device according to the first aspect of the present invention, the area of the culture well slope in respect of one or more of said well assemblies, preferably all well assemblies, is greater than the area of each associated washing well slope belonging to the same well assembly.

As it will primarily be the culture well which is used for accommodating a live biological material it will be beneficial for space economic reason to provide the area of a culture well slope with a greater area compared to the area of the associated washing well slope belonging to the same well assembly.

In one embodiment of the device according to the first aspect of the present invention, the angle of the culture well slope in relation to the horizontal plane in respect of one or more of said well assemblies, preferably all well assemblies, is 20 - 50°, such as 22 - 48°, for example 24 - 46°, e.g. 26 - 44°, such as 28 - 42°, e.g. 30 - 40°, such as 32 - 48 or 34 - 36°.

An angle lying within these ranges have proven it much easier for a laboratory technician to handle the pipetting work associated with change of culture medium etc. in the situation of culturing an embryo.

In one embodiment of the device according to the first aspect of the present invention, part of the rim of one or more of said well depressions in respect of one or more of said well assemblies, preferably all well assemblies, in an area opposite to the well slope, defines the beginning of a wall extending upwards, thus defining a well wall.

In one embodiment of the device according to the first aspect of the present invention, the angle of the well wall in relation to the horizontal plane is 52 - 90°, such as 54 - 88°, e.g. 56 - 86°, such as 58 - 84°, for example 60 - 82°, e.g. 62 - 80°, such as 64 - 78°, such as 66 - 76°, for example 68 - 74°, e.g. 70 - 72°.

It will thus be preferred that the angle of a well slope is steeper in relation the horizontal plane than the angle of a well wall in relation the horizontal plane.

It will also be preferred that one or more of the well walls are arranged in close proximity to its associated well depression rim.

Providing the device with a well wall extending from one part of the well rim opposite to the well slope having the above defined angles, has proven efficient in respect of saving space on the surface of the device. In this way a large number of well assemblies can be provided on a single device.

The rim of the culture well and optionally also the rim of the washing well is preferably circularly shaped having a diameter of 2.0 - 4.0 mm, such as 2.2 - 3.8 mm, for example 2.4 - 3.6 mm, e.g. 2.6 - 3.4 mm, for example 2.8 - 3.2 mm.

In one embodiment of the device according to the first aspect of the present invention, the dish additionally comprises a reservoir for accommodating a growth medium for biological matter.

Providing the device with a reservoir for accommodating a growth medium avoids the necessity of providing a culture well comprising a biological material with a pH probe, which in turn could cause mechanical damage to the biological material.

The reservoir may furthermore be provided with a lid. Such a lid is preferably made from a gas permeable material, such as silicone, e.g. PDMS silicone.

In one embodiment of the device according to the first aspect of the present invention, said device additionally comprises a lid adapted to be able to cover all well assemblies.

Providing the device with a lid will minimize the risk of contaminating any of the wells of the device, accommodating biological material with foreign matter.

In one embodiment of the device according to the first aspect of the present invention, the dish in its top surface comprises a cut-out, said cut-out is having an extension in the horizontal direction which is smaller than the extension of the dish itself, wherein said device additionally comprises a lid adapted to be able to cover all well assemblies, wherein said lid is adapted to detachable fit into this cut-out in the top surface of the dish in a locked configuration.

In such an embodiment it will be avoided that a laboratory technician by accident lifts the device in its lid, whereafter the device itself may be dropped and result in damages and intermixing of the cultured biological material.

In one embodiment of the device according to the first aspect of the present invention, said lid is made of a silicone material, such as PDMS.

In one embodiment of the device according to the first aspect of the present invention, one or more ventilation holes are provided in the dish, thus providing access for gas to enter the space above the well assemblies and below the lid, when the lid is attached to the dish in a locked configuration.

### The second aspect of the present invention

The second aspect of the present invention relates to a kit of parts comprising one or more devices according to the first aspect of the present invention; and an incubator, wherein said incubator is configured to accommodate one or more of said devices.

### The third aspect of the present invention

The third aspect of the present invention relates to the use of a device according to the first aspect, or of a kit of parts according to the second aspect for culturing a biological material.

In one embodiment the use according to the third aspect of the present invention, is for culturing an embryo or one or more stem cells.

In one embodiment the use according to the third aspect of the present invention, the embryo or the one or more stem cells are of a mammal origin, such as human being origin.

The device according to the first aspect of the present invention is especially adapted to be used for such purposes.

### The fourth aspect of the present invention

The fourth aspect of the present invention relates to a method of culturing a biological material, said method comprises:
- providing a device according to the first aspect of the present invention;
- arranging a biological material in the culture well depression of one or more of said culture wells;
- adding a growth medium to said culture well depressions;
- optionally covering said biological material and growth medium with an inert fluid, such as mineral oil;
- providing viable growth conditions to said biological material;
- allowing the biological material to grow for a predetermined time.

In one embodiment the method according to the fourth aspect of the present invention, the growth of the biological material is taking place in an incubator.

In one embodiment the use according to the fourth aspect of the present invention, the growth of the biological material is monitored, such as monitoring by a camera system.

In one embodiment the use according to the third aspect of the present invention, the growth conditions are controlled, such as by performing temperature control, performing pH control of growth medium, performing control of the gas surrounding the biological material.

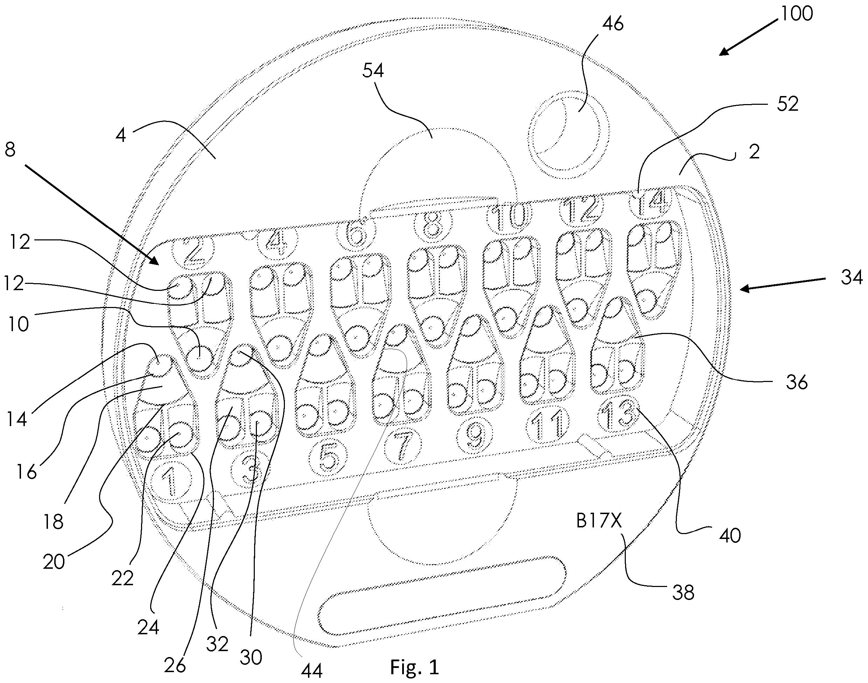

Referring now in details to the drawings for the purpose of illustrating preferred embodiments of the present invention, a device for culturing biological material according to the first aspect of the present invention is shown in a perspective view in fig. 1. Fig. 1 shows one embodiment of a device 100 for culturing biological material according to the first aspect of the present invention.

The device comprises a dish 2 made from a material and having a top surface 4 and a bottom surface 6 (not shown in fig. 1). The dish is provided with 14 well assemblies 8, which are numbered by well identification tags 40 by the numerals 1 - 14. Each well assembly 8 comprises a culture well 10 and two associated washing wells 12.

Each culture well comprises a culture well depression 14 surrounded by a culture well rim 16. Part of the culture well rim defines the beginning of a culture well slope 18 extending upward at an angle to a well slope plateau 20 of said culture well.

Likewise each washing well comprises a washing well depression 22 surrounded by a washing well rim 24. Part of the washing well rim 24 defines the beginning of a washing well slope 26 extending upward at an angle to a well slope plateau 28 of said washing well. In the embodiment shown in fig. 1, in respect of each well assembly, the washing well slope plateau 28 is shared with the associated culture well slope plateau 20 as a common slope plateau 36.

The washing well slope plateau 28 and the associated culture well slope plateau 20, and hence also the common slope plateau 36 is slightly lowered in relation to the top surface of the material of the dish. This feature will prevent that any culture medium upon being handled by a laboratory technician will migrate from one well assembly to another and accordingly enhanced the quality of the outcome of the work performed with the device.

Fig. 1 also shows a culture medium reservoir 46 arranged outside the area of the well assemblies. The culture medium reservoir is in use intended for accommodating a pH probe. Preferably the reservoir 46 will be covered by a lid (not shown). Preferably the lid will be permeable for gases, especially carbon dioxide. By providing a device according to the first aspect of the present invention with a culture medium reservoir it will possible to monitor the pH of the culture medium as a response to the "gas atmosphere" above the dish; and moreover this will be possible without the necessity to install a pH probe in a culture well accommodating biological matter. Ensuring separation of the pH probe and the biological matter will limit any mechanical damages on the biological matter otherwise imposable by such probe. Furthermore, any contamination from such probe to the biological material may be avoided.

Fig 1 furthermore shows a cut-out 52 for a lid. As it can be seen, the cut-out 52 for the lid (not shown), and thus also the lid (not shown) will have an extension in the horizontal plane not exceeding the physical dimension of the dish. The lid is designed to be pressed and locked into position in the cut-out 52. This design is an alternative to the prior art designs in which the lid covers the whole extension in a horizontal plane of the dish. However, in such a prior art design, a laboratory technician may easily in handling the device grasp and lift the device in its lid and subsequently drop the device.

In the design shown in fig. 1 a more save handling of the device by a laboratory technician will be ensured in that the lid may be locked into a locked configuration and accordingly no accidents are involved in simply grapping the lid when moving the device around.

In the event a biological material is incubated in a device according to the first aspect of the present invention in a situation in which the device is covered with a lid, it will be of paramount importance that the biological material has access to a desired atmosphere of gas. To this end, the device is provided with ventilation holes 54 as seen in fig. 1.

The device in fig. 1 is furthermore provided with a dish identification tag 38 enabling laboratory technician to distinguish one dish from the others.

It will be an advantage that as many as possible, preferably all culture well depressions are aligned on a straight line lying in a horizontal direction as seen in fig. 1. Such a design makes it possible for a camera in an incubator to only need to move in one direction in the process of acquisition of time lapse images.

It is seen in fig. 1 that the in respect of each well assembly, the area of the each washing well slope is smaller than the area of the associated culture well slope.

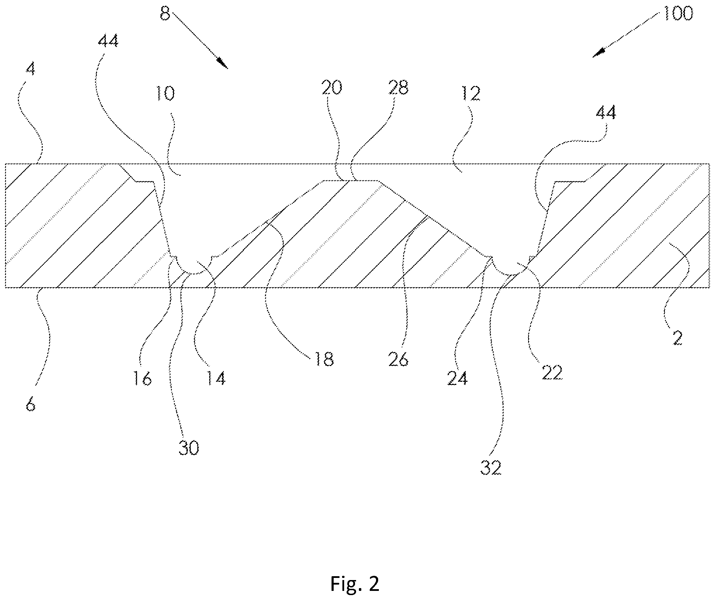

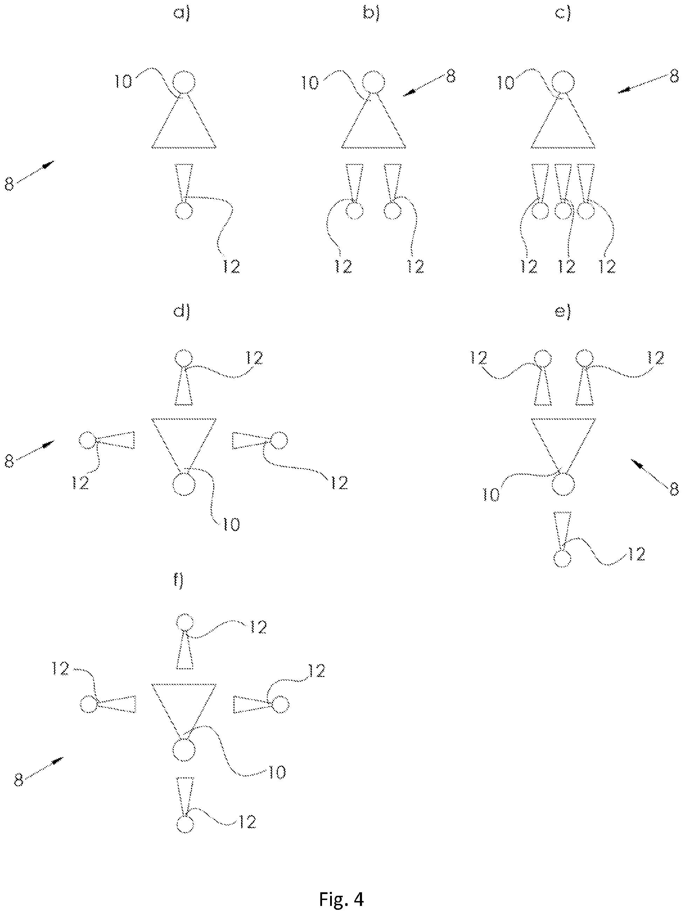

Fig. 2 shows a close-up of a cross-sectional view of a well assembly comprising a culture well and one single washing well in a configuration as the one depicted in fig. 4a.

Fig. 2 accordingly shows a well assembly 8 of a device 100. The well assembly comprises a culture well 10 and washing well 12. The culture well comprises a culture well depression 14 comprising a culture well depression rim 16. Part of the culture well depression rim 16 defines the beginning of a culture well slope 18 extending upward to a culture well slope plateau 20.

In the other direction of the well slope 18 of the culture well 10, part of the rim 16 of the culture well depression 14 defines the beginning of a culture well wall 44, having a steeper angle in relation to the horizontal direction, compared to the culture well slope 18.

The washing well shown in fig. 2 comprises a washing well depression 22 comprising a washing well depression rim 24. Part of the washing well depression rim 24 defines the beginning of a washing well slope 26 extending upward to a washing well slope plateau 28. The culture well slope plateau 20 and the washing well slope plateau 28 is defined by the same area.

In the other direction of the well slope 26 of the washing well 12, part of the rim 24 of the washing well depression 22 defines the beginning of a washing well wall 44, having a steeper angle in relation to the horizontal direction, compared to the washing well slope 26.

Also seen in fig. 2 is the bottom of the culture well depression 30 and the bottom of the washing well depression 32. These parts of the material of the dish are preferably transparent allowing a camera located below the dish to record images through said bottoms 30,32 of a biological material accommodated in the wells depressions 14, 22.

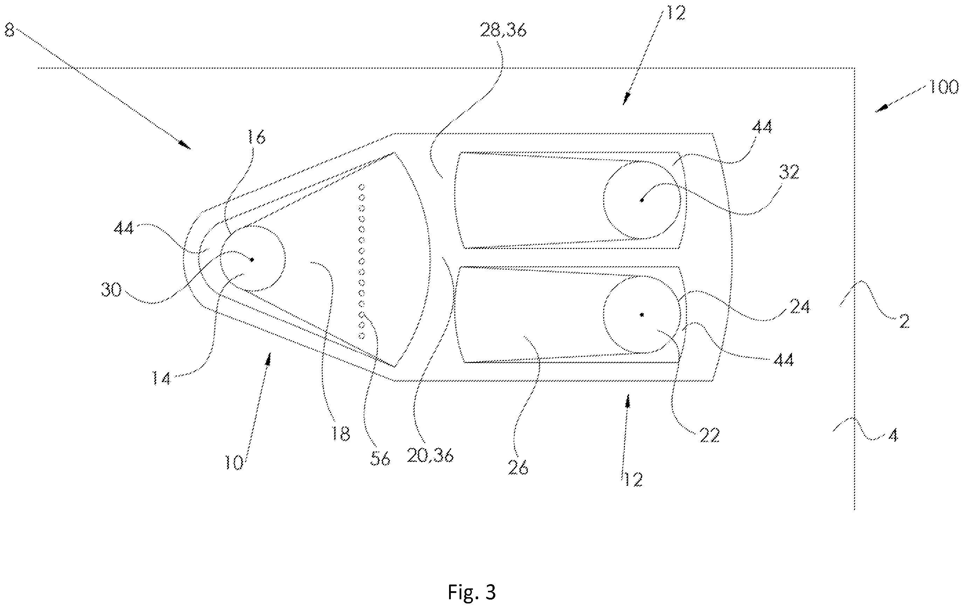

Fig. 3 shows a close-up plan view of a well assembly as shown in fig. 1. Fig 2 shows the well assembly 8 comprising a culture well 10 and two washing wells 12 accommodated in the top surface 4 of a dish 2 of a device 100. The culture well comprises a culture well depression 14, which in turn comprises a culture well depression rim 16. Part of the culture well depression rim 16 defines the beginning of a culture well slope extending upward to a well plateau 20,28,36 which is shared with the well plateau 28 of the washing wells. Fig. 3 also shows that in the opposite direction of the culture well slope 18, the rim 16 of the culture well depression 14 defines the beginning of a well wall 44. The culture well wall 44 has a much steeper slope than the culture well slope 18.

The washing wells each comprise a washing well depression 22, which in turn comprises a washing well depression rim 24. Part of the washing well depression rim 24 defines the beginning of a washing well slope extending upward to a well plateau 20,28,36 which is shared with the well plateau 20 of the culture well. Fig. 3 also shows that in the opposite direction of the washing well slope 26, the rim 24 of the washing well depression 22 defines the beginning of a well wall 44. The washing well wall 44 has a much steeper slope than the culture well slope 18.

The culture well, the washing wells, and the well slope plateau as well as the area in immediate vicinity of the wells are arranged slightly lowered, compared to the top surface 4 of the dish 2 of the device 100.

Fig. 3 shows that the culture well slope 18 is provided with a range of protrusions 56 arranged on a line across said culture well slope. Such protrusions may act as a filter filtering off any solids present in the culture medium added to the culture well. By adding the culture medium to the culture well at a position on the slope 18 being higher than the position of the protrusions, the culture medium will flow down towards the culture well 14 and any un-dissolved matter, such as solid contaminations in the culture medium will be stopped by the protrusions 56.

For the sake of simplicity these protrusions are not shown in fig. 1 and 2 and are not shown in fig. 4 neither.

Fig 4 is a plan view depicting various possible configurations of the well assemblies of the device according to the first aspect of the present invention. In fig. 4 a culture well is represented by a relatively large triangle with a circle at its apex, whereas a washing well is represented by a relatively small triangle with a circle at its apex.

The well assemblies according to the device of the first aspect of the present invention may comprises one culture well and one washing well. These may be arranged as depicted in fig. 1a.

Alternatively, the well assemblies according to the device of the first aspect of the present invention may comprises one culture well and two washing wells. These may be arranged as depicted in fig. 1b.

Still alternatively, the well assemblies according to the device of the first aspect of the present invention may comprises one culture well and three washing wells. These may be arranged as depicted in fig. 1c, 1d, or 1e.

The well assemblies according to the device of the first aspect of the present invention may also as a still further alternative comprise one culture well and four washing wells. Such a well assembly is depicted in fig. 1f).

It will be appreciated by those skilled in the art that changes could be made to the embodiments described above without departing from the broad inventive concept thereof. It is understood, therefore, that this invention is not limited to the particular embodiments disclosed, but it is intended to cover modifications within the spirit and scope of the present invention as defined by the appended claims.

### List of reference numerals

- 2: Dish
- 4: Top surface of dish
- 6: Bottom surface of dish
- 8: Well assembly
- 10: Culture well
- 12: Washing well
- 14: Culture well depression
- 16: Rim of culture well depression
- 18: Culture well slope
- 20: Well slope plateau of culture well
- 22: Washing well depression
- 24: Washing well rim
- 26: Washing well slope
- 28: Well slope plateau of washing well
- 30: Bottom of culturing well depression
- 32: Bottom of washing well depression
- 34: Straight line aligning depressions of culture well
- 36: Common slope plateau
- 38: Dish identification tag
- 40: Well assembly identification tag
- 42: Culture well identification tag
- 44: Well wall
- 46: Reservoir
- 48: Biological matter
- 50: Lid of device
- 52: Cut-out for lid
- 54: Ventilation holes
- 56: Filter protrusions of slope
- 100: Device
- 200: Kit of parts
- 300: Incubator

## Claims

1. A device (100) for culturing biological matter, said device in the orientation intended for use comprises:
- a dish (2) of a material, said dish having a top surface (4) and an essentially flat, horizontal bottom surface (6);
wherein said dish comprising a number of well assemblies (8), said number of well assemblies being 3 or more;
wherein in respect of one or more of said well assemblies, preferably all well assemblies, said well assembly comprising a culture well (10) and one or more associated washing wells (12),
wherein in respect of one or more of said well assemblies, preferably all well assemblies, said culture well and said one or more associated washing wells are grouped together;
wherein in respect of one or more of said well assemblies, preferably all well assemblies, said culture well comprises a culture well depression (14) in said material, said culture well depression having a rim (16), wherein at least one part of said rim defines the beginning of a slope extending upwards, thus defining a culture well slope (18), said culture well slope ending in a well slope plateau (20);
wherein in respect of one or more of said well assemblies (8), preferably all well assemblies, said washing well comprises a washing well depression (22) in said material, said washing well depression (22) having a rim (24), wherein at least one part of said rim defines the beginning of a slope extending upwards, thus defining a washing well slope (26), said washing well slope ending in a well slope plateau (28);
wherein in respect of one or more of said well assemblies (8), preferably all well assemblies, said material at the position of the bottom (30) of the culture well depression, and optionally also at the position of the bottom (32) of the washing well depression, being transparent
**characterized in that** in respect of one or more of the well assemblies, preferably all well assemblies, said culture well slope comprises a number of protrusions (56), protruding from the surface of the culture well slope, said number of protrusions being present across the slope in at least a part thereof, preferably in a horizontal direction.

2. A device according to claim 1, wherein the number of well assemblies (8) is 4 - 30, such as 6 - 28, e.g. 8 - 26, such as 10 - 24, for example 12 - 22, such as 14 - 20, e.g. 16 - 18.

3. A device according to any of the claims 1 - 2, wherein the number of washing wells (12) in each specific well assembly being 1, 2, 3 or 4.

4. A device according to any of the previous claims, wherein more than half of the culture well depressions (14) are arranged in such a way that they form a single straight line (34) extending in an essentially horizontal direction.

5. A device according to claim 4, wherein all the culture well depressions (14) are arranged in such a way that they form a single straight line (34) extending in an essentially horizontal direction.

6. A device according to claim 4 or 5, wherein the number of culture well depressions (14) arranged in a single straight line extending in an essentially horizontal direction is 3 - 20, such as 4 - 19, for example 5 - 18, such as 6 - 17, e.g. 7 - 16, for example 8 - 15, e.g. 9 - 14, such as 10 - 13 or 11 - 12.

7. A device according to any of the previous claims, wherein in respect of one or more of said well assemblies (8), preferably in respect of all well assemblies, the culture well depression (14) is having an essentially conical shape or an essentially spherical shape.

8. A device according to any of the previous claims, wherein in respect of one or more of said well assemblies (8), preferably in respect of all well assemblies, said culture well (10) and said one or more associated washing wells (12) are grouped together in a distinct group, such as being arranged more proximate to each other compared to any surrounding well.

9. A device according to any of the previous claims, wherein one or more, preferably all well assemblies comprises a well assembly identification tag (40).

10. A device according to any of the preceding claims, wherein in respect of one or more of said well assemblies (8), preferably all well assemblies, the angle of the culture well slope in relation to the horizontal plane is 20 - 50°, such as 22 - 48°, for example 24 - 46°, e.g. 26 - 44°, such as 28 - 42°, e.g. 30 - 40°, such as 32 - 48 or 34 - 36°.

11. A device according to any of the preceding claims, wherein the dish additionally comprises a reservoir (46) for accommodating a growth medium for biological matter (48).

12. A device according to any of the preceding claims, wherein said dish (2) in its top surface (4) comprises a cut-out (52), said cut-out is having an extension in the horizontal direction which is smaller than the extension in a horizontal direction of the dish itself, wherein said device additionally comprises a lid (50) adapted to be able to cover all well assemblies, wherein said lid is adapted to detachable fit into this cut-out (52) in the top surface of the dish in a locked configuration.

13. A device according to claim 12, wherein said lid is made of a silicone material, such as PDMS.

14. A kit of parts (200) comprising one or more of a device (100) according to any of the claims 1 - 13; and an incubator (300), wherein said incubator is configured to accommodate one or more of said devices.

15. Use of a device (100) according to any of the claims 1 - 13, or of a kit of parts (200) according to claim 14 for culturing a biological material.

16. Use according to claim 15 for culturing an embryo or one or more stem cells.

17. A method of culturing a biological material, said method comprises:
- providing a device (100) according to any of the claims 1 - 13;
- arranging a biological material in the culture well depression (14) of one or more of said culture wells (10);
- adding a growth medium to said culture well depressions;
- optionally covering said biological material and growth medium with an inert fluid, such as mineral oil;
- providing viable growth conditions to said biological material;
- allowing the biological material to grow for a predetermined time.

18. A method according to claim 17, wherein the growth of the biological material is monitored, such as monitoring by a camera system.

19. A method according to any of the claims 17 or 18, wherein the growth conditions are controlled, such as by performing temperature control, performing pH control of growth medium, performing control of the gas surrounding the biological material.

## Patentansprüche

1. Vorrichtung (100) zum Kultivieren von biologischem Material, wobei die Vorrichtung in der zur Verwendung vorgesehenen Orientierung aufweist:
- eine Schale (2) aus einem Material, die Schale aufweisend eine Oberseite (4) und eine im Wesentlichen flache, horizontale Bodenfläche (6);
wobei die Schale eine Anzahl von Wannenanordnungen (8) umfasst, wobei die Anzahl von Wannenanordnungen 3 oder mehr beträgt;
wobei in Bezug auf eine oder mehrere der Wannenanordnungen, vorzugsweise alle Wannenanordnungen, die Wannenanordnung eine Kulturwanne (10) und eine oder mehrere zugeordnete Waschwannen (12) umfasst,
wobei in Bezug auf eine oder mehrere der Wannenanordnungen, vorzugsweise alle Wannenanordnungen, die Kulturwanne und die eine oder mehrere assoziierte Waschwannen zusammen gruppiert sind;
wobei in Bezug auf eine oder mehrere der Wannenanordnungen, vorzugsweise alle Wannenanordnungen, die Kulturwanne eine Kulturwannenvertiefung (14) in dem Material umfasst, wobei die Kulturwannenvertiefung einen Rand (16) aufweist, wobei mindestens ein Teil von dem Rand den Anfang von einem Anstieg definiert, welche sich nach oben erstreckt, somit definierend einen Kulturwannenanstieg (18), wobei der Kulturwannenanstieg endet in einem Wannenanstiegsplateau (20);
wobei in Bezug auf eine oder mehrere der Wannenanordnungen (8), vorzugsweise alle Wannenanordnungen, die Waschwanne eine Waschwannenvertiefung (22) in dem Material umfasst, die Waschwannenvertiefung (22) aufweisend einen Rand (24), wobei mindestens ein Teil von dem Rand den Anfang von einem Anstieg definiert, welcher sich nach oben erstreckt, somit definierend eine Waschwannenanstieg (26), wobei der Waschwannenanstieg endet in einem Wannenanstiegsplateau (28);
wobei in Bezug auf eine oder mehrere der Wannenanordnungen (8), vorzugsweise alle Wannenanordnungen, das Material an der Position des Bodens (30) der Kulturwannenvertiefung, und optional auch an der Position des Bodens (32) der Waschwannenvertiefung, transparent ist
**dadurch gekennzeichnet, dass** in Bezug auf eine oder mehrere der Wannenanordnungen, vorzugsweise alle Wannenanordnungen, die Kulturwannenanstieg eine Anzahl von Vorsprüngen (56) umfasst, vorspringend von der Oberfläche von dem Kulturwannenanstieg, wobei die Anzahl von Vorsprüngen anwesend sind quer über den Anstieg in mindestens einem Teil davon, vorzugsweise in einer horizontalen Richtung.

2. Vorrichtung nach Anspruch 1, wobei die Anzahl der Wannenanordnungen (8) 4 - 30 beträgt, wie 6 - 28, z. B. 8 - 26, wie 10 - 24, zum Beispiel 12 - 22, wie 14 - 20, z. B. 16 - 18.

3. Vorrichtung ach einem der Ansprüche 1 - 2, wobei die Anzahl der Waschwannen (12) in jeder spezifischen Wannenanordnung 1, 2, 3 oder 4 beträgt.

4. Vorrichtung nach irgendeinem den vorhergehenden Ansprüchen, wobei mehr als die Hälfte der Kulturwannenvertiefungen (14) auf solche Art und Weise angeordnet sind, dass sie eine einzelne gerade Linie (34) bilden, welche sich in einer im Wesentlichen horizontalen Richtung erstreckt.

5. Vorrichtung nach Anspruch 4, wobei alle Kulturwannenvertiefungen (14) auf solche Art und Weise angeordnet sind, dass sie eine einzelne gerade Linie (34) bilden, welche sich in einer im Wesentlichen horizontalen Richtung erstreckt.

6. Vorrichtung nach Anspruch 4 oder 5, wobei die Anzahl der Kulturwannenvertiefungen (14), angeordnet in einer einzelnen geraden Linie, welche sich in einer im Wesentlichen horizontalen Richtung erstreckt, 3 - 20 beträgt, wie 4 - 19, zum Beispiel 5 - 18, wie z 6 - 17, z. B. 7 - 16, zum Beispiel 8 - 15, z. B. 9 - 14, wie 10 - 13 oder 11 - 12.

7. Vorrichtung nach einem der vorhergehenden Ansprüche, wobei in Bezug auf eine oder mehrere von den Wannenanordnungen (8), vorzugsweise in Bezug auf alle Wannenanordnungen, die Kulturwannenvertiefung (14) eine im Wesentlichen konische Form oder eine im Wesentlichen sphärische Form aufweist.

8. Vorrichtung nach einem der vorhergehenden Ansprüche, wobei in Bezug auf eine oder mehrere der Wannenanordnungen (8), vorzugsweise in Bezug auf alle Wannenanordnungen, die Kulturwanne (10) und die eine oder mehrere assoziierte Waschwannen (12) zusammen gruppiert sind in einer bestimmten Gruppe, wie zum Beispiel mehr zueinander angeordnet verglichen zu irgendeiner umgebenden Wanne.

9. Vorrichtung nach einem der vorhergehenden Ansprüche, wobei eine oder mehrere, vorzugsweise alle Wannenanordnungen, eine Wannenanordnungen-Identifikationsmarke (40) umfassen.

10. Vorrichtung nach einem der vorhergehenden Ansprüche, wobei in Bezug auf eine oder mehrere der Wannenanordnungen (8), vorzugsweise alle Wannenanordnungen, der Winkel von dem Kulturwannenanstieg in Relation zu der horizontalen Ebene 20 - 50° beträgt, wie 22 - 48°, zum Beispiel 24 - 46°, z. B. 26 - 44°, wie 28 - 42°, z. B. 30 - 40°, wie 32 - 48 oder 34 - 36°.

11. Vorrichtung nach einem der vorhergehenden Ansprüche, wobei die Schale zusätzlich einen Vorratsbehälter (46) zur Aufnahme eines Wachstumsmediums für biologisches Material (48) umfasst.

12. Vorrichtung nach einem der vorhergehenden Ansprüche, wobei die Schale (2) in ihrer Oberseite einen Ausschnitt (52) umfasst, wobei der Ausschnitt eine Ausdehnung in der horizontalen Richtung aufweist, welche kleiner ist als die Ausdehnung in einer horizontalen Richtung von der Schale selbst, wobei die Schale zusätzlich einen Deckel (50) umfasst, adaptiert, um in der Lage zu sein, alle Wannenanordnungen zu bedecken, wobei der Deckel adaptiert ist, um lösbar in diesen Ausschnitt (52) in die Oberseite von der Schale in einer geschlossenen Konfiguration zu passen.

13. Vorrichtung nach Anspruch 12, wobei der Deckel aus einem Silikonmaterial, wie zum Beispiel PDMS, hergestellt ist.

14. Ein Bausatz von Teilen (200) umfassend eine oder mehrere einer Vorrichtung (100) nach einem der Ansprüche 1 - 13; und einen Inkubator (300), wobei der Inkubator konfiguriert ist, um eine oder mehrere der Vorrichtungen aufzunehmen.

15. Verwendung einer Vorrichtung (100) nach einem der Ansprüche 1-13 oder einem Bausatz von Teilen (200) nach Anspruch 14 zur Kultivierung eines biologischen Materials.

16. Verwendung nach Anspruch 15 zur Kultivierung eines Embryos oder einer oder mehrerer Stammzellen.

17. Verfahren zur Kultivierung eines biologischen Materials, wobei das Verfahren umfasst:
- Bereitstellen einer Vorrichtung (100) nach einem der Ansprüche 1 - 13;
- Anordnen eines biologischen Materials in der Kulturwannenvertiefung (14) von einer oder mehreren der Kulturwannen (10);
- Hinzugabe eines Wachstumsmediums zu den Kulturwannenvertiefungen;
- gegebenenfalls Abdecken des biologischen Materials und des Wachstumsmediums mit einem inerten Fluid, wie zum Beispiel Mineralöl;
- Bereitstellen lebensfähiger Wachstumsbedingungen zu dem biologische Material;
- Erlauben, dass das biologische Material für eine vorbestimmte Zeit wächst.

18. Ein Verfahren nach Anspruch 17, wobei das Wachstum des biologischen Materials überwacht wird, wie zum Beispiel Überwachen durch ein Kamerasystem.

19. Ein Verfahren nach einem der Ansprüche 17 oder 18, wobei die Wachstumsbedingungen kontrolliert werden, wie zum Beispiel durch Durchführen einer Temperatursteuerung, Durchführen einer pH-Steuerung des Wachstumsmediums, Durchführen einer Kontrolle des Gases, welches das biologische Material umgibt.

## Revendications

1. Dispositif (100) pour la culture de matière biologique, ledit dispositif placé selon l'orientation servant à l'utiliser comprend :
- une capsule (2) d'un matériel, ladite capsule possédant une surface supérieure (4) et une surface inférieure horizontale essentiellement plate (6) ;
dans lequel ladite capsule comprenant un certain nombre d'ensembles de puits (8), ledit nombre d'ensembles de puits étant 3 ou plus ;
dans lequel en ce qui concerne un ou plusieurs desdits ensembles de puits, de préférence la totalité des ensembles de puits, ledit ensemble de puits comprenant un puits de culture (10) et un ou plusieurs puits de lavage associés (12),
dans lequel en ce qui concerne un ou plusieurs desdits ensembles de puits, de préférence la totalité des ensembles de puits, ledit puits de culture et lesdits un ou plusieurs puits de lavage associés sont regroupés ensemble ;
dans lequel en ce qui concerne un ou plusieurs desdits ensembles de puits, de préférence la totalité des ensembles de puits, ledit puits de culture comprend un évidement de puits de culture (14) dans ledit matériel, ledit évidement de puits de culture possédant un bord (16), dans lequel au moins une partie dudit bord définit le début d'une pente s'étendant vers le haut, définissant ainsi une pente de puits de culture (18), ladite pente de puits de culture se terminant en un plateau de pente de puits (20) ;
dans lequel en ce qui concerne un ou plusieurs desdits ensembles de puits (8), de préférence la totalité des ensembles de puits, ledit puits de lavage comprend un évidement de puits de lavage (22) dans ledit matériel, ledit évidement de puits de lavage (22) possédant un bord (24), dans lequel au moins une partie dudit bord définit le début d'une pente s'étendant vers le haut, définissant ainsi une pente de puits de lavage (26), ladite pente de puits de lavage se terminant en un plateau de pente de puits (28) ;
dans lequel en ce qui concerne un ou plusieurs desdits ensembles de puits (8), de préférence la totalité des ensembles de puits, ledit matériel à la position du fond (30) de l'évidement de puits de culture, et optionnellement également à la position du fond (32) de l'évidement de puits de lavage, étant transparent
**caractérisé en ce que** en ce qui concerne un ou plusieurs des ensembles de puits, de préférence la totalité des ensembles de puits, ladite pente de puits de culture comprend un certain nombre de saillies (56), faisant saillie à partir de la surface de la pente de puits de culture, ledit nombre de saillies étant présent à travers la pente dans au moins une partie de celle-ci, de préférence dans une direction horizontale.

2. Dispositif selon la revendication 1, dans lequel le nombre d'ensembles de puits (8) est 4 à 30, par exemple 6 à 28, par exemple 8 à 26, par exemple 10 à 24, par exemple 12 à 22, par exemple 14 à 20, par exemple 16 à 18.

3. Dispositif selon l'une quelconque des revendications 1 à 2, dans lequel le nombre de puits de lavage (12) dans chaque ensemble spécifique de puits étant 1, 2, 3 ou 4.

4. Dispositif selon l'une quelconque des revendications précédentes, dans lequel plus de la moitié des évidements de puits de culture (14) sont agencés de manière telle qu'ils forment une seule ligne droite (34) s'étendant dans une direction essentiellement horizontale.

5. Dispositif selon la revendication 4, dans lequel tous les évidements de puits de culture (14) sont agencés de manière telle qu'ils forment une seule ligne droite (34) s'étendant dans une direction essentiellement horizontale.

6. Dispositif selon la revendication 4 ou 5, dans lequel le nombre d'évidements de puits de cultures (14) agencés en une seule ligne droite s'étendant dans une direction essentiellement horizontale est 3 à 20, par exemple 4 à 19, par exemple 5 à 18, par exemple 6 à 17, par exemple 7 à 16, par exemple 8 à 15, par exemple 9 à 14, par exemple 10 à 13 ou 11 à 12.

7. Dispositif selon l'une quelconque des revendications précédentes, dans lequel en ce qui concerne un ou plusieurs desdits ensembles de puits (8), de préférence en ce qui concerne la totalité des ensembles de puits, l'évidement de puits de culture (14) présente une forme essentiellement conique ou une forme essentiellement sphérique.

8. Dispositif selon l'une quelconque des revendications précédentes, dans lequel en ce qui concerne un ou plusieurs desdits ensembles de puits (8), de préférence en ce qui concerne la totalité des ensembles de puits, ledit puits de culture (10) et lesdits un ou plusieurs puits de lavage associés (12) sont regroupés ensemble en un groupe distinct, par exemple agencés plus à proximité les uns des autres par rapport à un quelconque puits environnant.

9. Dispositif selon l'une quelconque des revendications précédentes, dans lequel un ou plusieurs, de préférence la totalité, des ensembles de puits comprennent une étiquette d'identification d'ensemble de puits (40).

10. Dispositif selon l'une quelconque des revendications précédentes, dans lequel en ce qui concerne un ou plusieurs desdits ensembles de puits (8), de préférence la totalité des ensembles de puits, l'angle de la pente de puits de culture par rapport au plan horizontal est 20 à 50°, par exemple 22 à 48°, par exemple 24 à 46°, par exemple 26 à 44°, par exemple 28 à 42°, par exemple 30 à 40°, par exemple 32 à 48 ou 34 à 36°.

11. Dispositif selon l'une quelconque des revendications précédentes, dans lequel la capsule comprend en outre un réservoir (46) pour loger un milieu de croissance pour matière biologique (48).

12. Dispositif selon l'une quelconque des revendications précédentes, dans lequel ladite capsule (2) dans sa surface supérieure (4) comprend une découpe (52), ladite découpe possède une extension dans la direction horizontale qui est plus petite que l'extension dans une direction horizontale de la capsule elle-même, dans lequel ledit dispositif comprend en outre un couvercle (50) adapté pour être capable de couvrir la totalité des ensembles de puits, dans lequel ledit couvercle est adapté pour s'ajuster de façon détachable dans cette découpe (52) dans la surface supérieure de la capsule dans une configuration verrouillée.

13. Dispositif selon la revendication 12, dans lequel ledit couvercle est fait d'un matériel en silicone, par exemple PDMS.

14. Kit de pièces (200) comprenant un ou plusieurs d'un dispositif (100) selon l'une quelconque des revendications 1 à 13 ; et une étuve (300), dans lequel ladite étuve est configurée pour loger un ou plusieurs desdits dispositifs.

15. Utilisation d'un dispositif (100) selon l'une quelconque des revendications 1 à 13, ou d'un kit de pièces (200) selon la revendication 14 pour la culture d'un matériel biologique.

16. Utilisation selon la revendication 15 pour la culture d'un embryon ou d'une ou plusieurs cellules souches.

17. Procédé de culture d'un matériel biologique, ledit procédé comprend :
- la fourniture d'un dispositif (100) selon l'une quelconque des revendications 1 à 13;
- l'agencement d'un matériel biologique dans l'évidement de puits de culture (14) d'un ou plusieurs desdits puits de culture (10) ;
- l'ajout d'un milieu de croissance dans lesdits évidements de puits de culture ;
- optionnellement la couverture dudit matériel biologique et dudit milieu de croissance avec un fluide inerte, par exemple de l'huile minérale ;
- la fourniture de conditions de croissance viables audit matériel biologique ;
- le fait de permettre la croissance libre dudit matériel biologique pendant une période prédéterminée.

18. Procédé selon la revendication 17, dans lequel la croissance du matériel biologique est surveillée, par exemple la surveillance par un système de prise de vues.

19. Procédé selon l'une quelconque des revendications 17 ou 18, dans lequel les conditions de croissance sont contrôlées, par exemple en effectuant un contrôle de température, effectuant un contrôle de pH de milieu de croissance, effectuant un contrôle du gaz entourant le matériel biologique.
